Europäisches Patentamt

European Patent Office

Office européen des brevets

(19)

(11) Veröffentlichungsnummer: **0 242 827 B1**

(12) **EUROPÄISCHE PATENTSCHRIFT**

(45) Veröffentlichungstag der Patentschrift: 27.12.91

(51) Int. Cl.⁵: **A61M 37/00**

(21) Anmeldenummer: 87105745.1

(22) Anmeldetag: 16.04.87

(54) **Flächenförmiges therapeutisches System, Verfahren zu seiner Herstellung und seine Verwendung.**

(30) Priorität: 17.04.86 DE 3613002
06.10.86 DE 3634016

(43) Veröffentlichungstag der Anmeldung:
28.10.87 Patentblatt 87/44

(45) Bekanntmachung des Hinweises auf die
Patenterteilung:
27.12.91 Patentblatt 91/52

(84) Benannte Vertragsstaaten:
AT BE CH DE ES FR GB GR IT LI LU NL SE

(56) Entgegenhaltungen:
EP-A- 0 147 119
AT-B- 201 781
FR-A- 2 562 800
GB-A- 2 021 950
US-A- 3 993 072

(73) Patentinhaber: **Lohmann GmbH & Co. KG**
**Irlicher Strasse 55**
**W-5450 Neuwied 12(DE)**

Patentinhaber: **SANDOZ AG**
**Lichtstrasse 35**
**CH-4002 Basel(CH)**

(72) Erfinder: **Wolter, Karin, Dr.**
**Altwieder Strasse 46**
**W-5451 Melsbach(DE)**
Erfinder: **Herrmann, Fritz, Dr.**
**Rheinheldestrasse 12c**
**W-5450 Neuwied 12(DE)**
Erfinder: **Hoffmann, Rainer, Dr.**
**Burghofstrasse 113**
**W-5450 Neuwied 22(DE)**
Erfinder: **Simon, Günter**
**Tulpenweg 1**
**W-5533 Hillesheim(DE)**
Erfinder: **Kissel, Thomas, Dr.**
**Federerweg 10**
**W-7801 Ehrenkirchen 1(DE)**
Erfinder: **Reinhardt, Joerg, Dr.**
**Schauinslandstrasse 5**
**W-7801 Ehrenkirchen(DE)**

(74) Vertreter: **Neidl-Stippler, Cornelia, Dr.**
**Neidl-Stippler & Partner Rauchstrasse 2**
**W-8000 München 80(DE)**

**Beschreibung**

Die Erfindung betrifft ein flächenförmiges therapeutisches System zur Verabreichung von Wirkstoffen an die Haut, mit Wirkstoffreservoir(s) mit Wirkstoffabgabeflächen und mit hautseitig angeordneten Haftklebebereichen; ein Verfahren zur Herstellung des flächenförmigen, therapeutischen Systems sowie dessen Verwendung.

Die Erfindung bezieht sich also auf ein transdermales therapeutisches System zur Verabreichung medizinischer Wirkstoffe oder auch kosmetisch wirksamer Substanzen an die menschliche oder tierische Haut.

Ein therapeutisches System ist eine arzneistoff- bzw. wirkstoff-enthaltende Vorrichtung bzw. eine Darreichungsform, die einen oder mehrere Wirkstoffe in vorausbestimmter Rate kontinuierlich über einen festgelegten Zeitraum an einem festgelegten Anwendungsort abgibt.

Diese Systeme sind therapeutische Präzisionsinstrumente, deren Konstruktion außergewöhnliche Maßnahmen erfordern, um die kontinuierliche Wirkstoff-Freisetzung sicherzustellen.

Therapeutische Systeme sind schon für die verschiedensten Anwendungsorte entwickelt worden, so auch für die Haut, wobei neben topischer auch systemische Wirkung erzielt werden kann. Die Vielfalt der auf diese Weise applizierbaren Wirkstoffe und ihre unterschiedlichen chemischen, physikalischen und pharmakologischen Eigenschaften machen es unmöglich, mit nur einem System alle therapeutischen Problemstellungen abzudekken.

Es sind schon zahlreiche flächenförmiges therapeutische Systeme zur Verabreichung von medizinischen Wirkstoffen an die Haut bekannt geworden, wobei eine Zusammenfassung beispielsweise in Klaus Heilmann: Therapeutische Systeme, Ferdinand Enke Verlag, Stuttgart, 1977 zu finden ist. In nicht allen Fällen konnte durch die Systeme nach dem Stand der Technik eine völlig befriedigende Wirkung erzielt werden.

Dabei ist ein üblicher Aufbau eines derartigen transdermalen therapeutischen Systems ein Arzneistoffreservoir, in dem der Wirkstoff in fester, flüssiger oder gelöster Form vorliegt und eine druckempfindliche Adhäsionsschicht, durch die das System mit der Haut eng verbunden wird.

Dieses Prinzip findet dort seine Grenzen, wo der Wirkstoff nicht durch die Haftkleber-Schicht diffundiert oder aber eine chemische Reaktion zwischen Wirkstoff und Haftkleber auftritt, oder aber der Wirkstoff im Haftkleber nicht oder nur schlecht löslich ist. In diesen Fällen ist versucht worden, ein nicht haftklebendes wirkstoffhaltiges Reservoir oder den Wirkstoff selbst in unmittelbaren Kontakt mit der Haut zu bringen und dieses Reservoir oder den Wirkstoff selbst durch zusätzliche Maßnahmen auf der Haut zu fixieren. Dazu eigenen sich beispielsweise separate haftklebende Streifen oder die Integration des Reservoirs oder des Wirkstoffes in einem Pflaster derart, daß das Reservoir oder der Wirkstoff ringsum von einen haftklebenden Rand umgeben ist (s. bspw. DE-OS 29 02 183). Übersteigt die Kontaktfäche eine bestimmte Größe, ist der zur gesteuerten Therapie notwendige gleichbleibende Kontakt mit der Haut bei längerer Tragezeit, die bekanntlich Tage, ja Wochen betragen kann, wegen der unvermeidlichen Körper- und Muskelbewegungen nicht mehr gewährleistet.

In der DE-OS 32 02 775 ist nun vorgeschlagen worden, den Wirkstoff rasterartig auf eine Haftklebefläche zu verteilen. Die Klebefläche ist im Prinzip dann zur Fixierung des Wirkstoffes auf der Haut groß genug und sollte theoretisch eine-flächige Fixierung des Wirkstoff-Reservoirs ermöglichen. Da sich der Wirkstoff aber auf der Klebeschicht, nicht auf dem gleichen Niveau mit derselben, befindet, steht diese nach Ankleben auf die Haut ständig unter Spannung. Aus diesem Grunde lösen sich die verklebten Stellen bei den untervermeidlichen Körper- und Muskelbewegungen sehr leicht wieder ab, beeinträchtigen den gleichbleibenden Kontakt der Wirkstoff-Fläche mit der Haut durch auftretende Druckänderung und verhindern damit jegliche gesteuerte Abgabe des Wirkstoffes.

Aus der DE-OS 34 23 328 ist ein selbstklebendes Pflaster mit separatem Wirkstoffsegmenten bekannt geworden, bei dem kalottenförmige Wirkstoffsegmente und Klebstoffsegmente auf einer nicht klebenden Unterlage angeordnet sicn. Dabei wird angeregt, daß Klebstoffsegmente und Wirkstoffsegmente gleiche Höhe haben sollen. Bei Applikation des Pflasters hängt die Größe der Hautkontaktsegmente und damit die freigesetzte Wirkstoffmenge vom Druck auf das Pflaster ab. Als therapeutisches System ist dieses Pflaster unbrauchbar.

In der DE-OS 33 19 469 ist vorgeschlagen worden, den Wirkstoff in den Poren einer mikroporösen Folie zu deponieren, die zur Fixierung an der Haut partiell oder vollständig mit einem Haftkleber beschichtet sein kann. Auch bei dieser Ausführungsform ist der Kontakt der Wirkstoffe in den Porenöffnungen mit der Haut beeinträchtigt, da die Porenöffnungen nicht auf dem gleichen Niveau mit den Haftklebebereichen liegen und demzufolge ein Kontakt zwischen Wirkstoff und Hautoberfläche durch die Haftklebeschicht verhindert wird. Auch mit diesem System ist daher eine gesteuerte Verabreichung von Wirkstoffen unmöglich.

Ein guter flächiger Kontakt eines transdermalen Sytems auf der Haut ist aber wesentlich für seinen Einsatz, da ohne permanenten kontinuierlich gleichbleibenden Kontakt kein Wirkstoff geregelt auf die Haut übertragen werden kann.

Demgegenüber ist es Aufgabe der Erfindung, ein flächenförmiges therapeutisches System zur Verabreichung von Wirkstoffen an die Haut zu schaffen, das unabhängig von der notwendigen Größe der Wirkstoffabgabefläche auch bei längerer Tragzeit des Systems einen bleibenden Kontakt der Wirkstoffabgabefläche zur Haut sicherstellt.

Die Aufgabe wird bei einem aus der FR-A-2562800 bekannten flächenförmigen therapeutischen System mit Wirkstoffreservoir, Wirkstoffabgabeflächen und mit diesen auf gleichem Niveau liegender hautseitig angeordneter Hautadhäsionsfläche dadurch gelöst, daß entweder die Hautadhäsionsfläche in unterbrochener Weise über die gesamte Hautkontaktfläche verteilt ist und die Unterbrechungen durch Wirkstoffabgabeflächenabschitte gebildet sind oder die Wirkstoffabgabefläche in unterbrochener Weise über die gesamte Hautkontaktfläche verteilt ist und die Unterbrechungen durch Hautadhäsionsflächenabschnitte gebildet sind. Bei einer vorteilhaften Weiterbildung des Erfindungsgegenstandes sind die Wirkstoffabgabeflächenabschnitte gleichmäßig oder ungleichmäßig in der Hautkontaktfläche verteilt. Es kann besonders vorteilhaft sein, wenn die Wirkstoffabgabeflächenabschnitte rund oder eckig sind.

In der Hautkontaktfläche können Hautadhäsionsflächenabschnitte regelmäßig oder unregelmäßig verteilt sein. Die Hautadhäsionsflächenabschnitte können rund oder eckig sein. Es kann für bestimmte Anwendungsformen aber auch besonders bevorzugt sein, daß Hautadhäsions- und Wirkstoffabgabeflächenabschnitte streifenförmig alternierend angeordnet sind. Die Hautkontaktfläche kann bei einer besonders bevorzugten Ausgestaltung der Erfindung einen umlaufenden Hautadhäsionsrand aufweisen. Das erfindungsgemäße System kann ferner eine hautabgewandte flexible Rückschicht und gegebenenfalls eine ablösbare Schutzschicht aufweisen. Außerdem kann es zur Verbesserung der mechanischen Stabilität eine Öffnungen aufweisende Stützschicht besitzen. Die Stützschicht kann im wesentlichen aus Papier, einen textilen Flächengebilde, einer Metall- oder einer Kunststoff-Folie oder aus Laminaten derselben bestehen. Zwischen Stützschicht und Wirkstoffreservoir kann ganz oder teilweise eine Haftklebeschicht vorhanden sein. Das Wirkstoffreservoir kann einen oder meherer Wirkstoff(e) mit topischer oder systemischer Wirkung aufweisen,

Bevorzugt ist der/die Wirkstoffe ausgewählt aus der Gruppe der Herzglycoside aus Digitalis lanata-β-Acetyldigoxin; Vasodilatoren, z.B. Pentaerithrityltetranitrat, Cinnarizin oder Nitroglycerin, den musculotropen Spasmolytika, bspw. Moxaverin HCl; Coronartherapeutica, z.B. Oxyfedrin HCl, Coronardilatatoren, z.B. N-(3,3-Diphenylpropyl)-Alphamethyl-benzylamin HCl (Fendilin); Antihistaminica, wie Clemastin oder Antiemeticum-Dimethhydrinat, Analeptica, wie Coffein; Analgetica, wie Phenazon-Chloralhydrat; Hypnotica, wie Chlorobutanol, Musculotrope Vasodilatatoren, wie Nicotinsäure, Vitamin B6, wie Pyridoxin, Broncholytica, Cardiaca, Diuretica, Phosphordiesterasehemmer Theophillin und Verbindungen desselben,bspw. Cholin-Theophyllinat, Theophyllin-Ethylendiamin, Theophyllinnatriumglycinat, Theophyllinnatriumsalicylat; sowie Theophyllin-Derivate, Bromtheophyllin, Chlortheophyllin, Etaminphyllin, Diprophyllin Etofyllin und Poxyphyllin sowie Rubefazientes, wie Nicotinsäure-β-butoxyethylester und/oder Nonylsäurevanillylamid; Antiphlogistica, wie Ethylenglykolmonosalicylat oder Diethylaminsalicylat. Bevorzugte Wirkstoffe sind Amphetaminil, Bethahistin, Beta-Acetyldigoxin, Bopindolol, Buprenorphin, Clemastin, Diclofenac, Diltiazen, Dimenhydrinat, Diethylaminsalicylat, Ethylenglykolmonosalicylat, 5-Fluorouracil, Glibenclamid, Hydromorphon, Ibuprofen, Isopropyl-4-(2,1,3-benzoxadiazol-4-yl)-1,4-dihydro-5-methoxycarbonyl-2,6-dimethyl-3-pyridin-carboxylat, Ketotifen, L-Thyroxin, Nicotin, Nicotinsäure-βbutoxyethylester, Nonylsäurevanillylamid, Pindolol, Salbutamol, Tamoxifen, Tizanidin, sowie deren Basen, Salze und Derivate. Die Wirkstoffe können auch in sinnvoller Kombination miteinander eingesetzt werden, bevorzugt sind:

A. Herzglycosid aus Digitalis lanata-β-Acetyldigoxin in Kombination mit

-1. einem Vasodilatator, z.B. Pentaerithrityltetranitrat oder Nitroglycerin,

-2. einem musculotropen Spasmolyticum, z. B. Moxaverin HCl

-3. einem Coronartherapeuticum, Z.B. Oxyfedrin HCl oder

-4. einem Coronardilatator, z.B. N-(3.3-Diphenylpropyl)-Alpha-methyl-benzylamin HCl (Fendilin)

B. Antihistaminicum, Clemastin, in Kombination mit einem Glucocorticoid, z.B. Dexamethason oder Clocortolon-21-pivalat;

C. Antihistaminicum, wie Antiemeticum-Dimenhydrinat- in Kombination mit einem

-1. Vasodilatator, Antihistaminicum, z.B. Cinnarizin,

-2. Analepticum, z.B. Coffein,

-3. Analgeticum, Antipyreticum, z.B. Phenazon-Chloralhydrat;

-4. Hypnoticum, Anaestheticum, Antisepticum, z.B. Chlorobutanol;

-5. Musculotropen Vasodilatator, Lipidsenker, z.B. Nicotinsäure, oder

-6. Vitamin B6, z.B. Pyridoxin.

D. Broncholyticum, Cardiacum, Diureticum, Phophordiesterasehemmer - Theophyllin - in Kombination mit anderen

-1. Theophyllin-Verbindungen, wie:

Cholin-Theophyllinat
Theophyllin-Ethylendiamin
Theophyllinnatriumglycinat
Theophyllinnatriumsalicylat, oder
-2. Theophyllin-Derivaten, wie:
Bromtheophyllin
Chlortheophyllin
Etaminphyllin
Diprophyllin
Etofyllin
Proxyphyllin

E. Rubefazientes, wie Nicotinsäure-$\beta$-butoxyethylester und/oder Nonylsäurevanillylamid - in Kombination mit einem Antiphlogisticum, z.B. Ethylenglykolmonosalicylat oder Diethylaminsalicylat.

Ein besonderer durch derartige Wirkstoff-Kombinationen erzielbarer Vorteil liegt darin, daß idealerweise mehrere Wirkstoffe gleichzeitig, ggf. mit unterschiedlichen Abgabegeschwindigkeiten, verabreicht werden können, die nicht in Mischung - sei es wegen Reaktionen untereinander oder unterschiedlichen physikalischen Eigenschaften verarbeitet oder gelagert werden können. Die später erläuterten Herstellungsschritte lassen problemlos eine getrennte Einarbeitung der Wirkstoffe zu.

Besonders bevorzugte, im erfindungsgemäßen System einsetzbare Wirkstoffe sind:
Amphetaminil, Bethahistin, $\beta$-Acetyldigoxin, Bopindolol, Buprenorphin, Clemastin, Diclofenac, Diltiazen, Dimenhydrinat, Diethylaminsalicylat, Ethylenglykol-monosalicylat, 5-Fluorouracil, Glibenclamid, Hydromorphon, Ibuprofen, Isopropyl-4-(2,1,3-benzoxadiazol-4-yl)-1,4-dihydro-5-methoxy-carbonyl-2,6-dimethyl-3-pyridin-carboxylat, Ketotifen, L-Thyroxin, Nicotin, Nicotinsäure-$\beta$-butoxyethylester, Nonylsäurevanillylamid, Pindolol, Salbutamol, Tamoxifen, Tizanidin, Theophyllin, sowie die Basen, Salze und Derivate der vorgenannten bevorzugten Wirkstoffe.

Die Erfindung bezieht sich auch auf Verfahren zur Herstellung des flächenförmigen therapeutischen Systems, das folgende Schritte aufweist:

Vorlegen einer abweisenden Unterlage; Erzeugen einer Hautadhäsionsschicht auf der Unterlage. Zukaschieren eines Stützmaterials; Perforation des derart gebildeten Laminats, Austausch der abweisenden Unterlage gegen eine ablösbare Schutzschicht; Beschichtung der schutzschichtfreien Oberfläche mit wirkstoffhaltiger Reservoirmasse; Zukaschieren der ggf. haftklebend ausgerüsteten Rückschicht und Konfektionierung.
Bei einer besonders bevorzugten Weiterbildung dieses Verfahrens wird nach der Perforation des Laminats eine Beschichtung mit der wirkstoffhaltigen Reservoirmasse durch Zukaschieren mit einer auf der Rückschicht erzeugten wirkstoffhaltigen Reservoirschicht unter Druck oder unter Druck/Wärme durchgeführt.

Bei einem weiteren besonders bevorzugten Verfahren werden Hautadhäsionsabschnitte auf eine Schutzschicht-Unterlage aufgebracht, diese Hautadhäsionsschichtabschnitte und die Schutzschichtunterlate mit wirkstoffhaltiger Reservoirmasse beschichtet, eine ggf. haftklebend ausgerüstete Rückschicht zukaschiert und das fertige System konfektioniert

Die Erfindung befaßt sich auch mit der Verwendung des Systems für die lokale oder systemische transdermale Wirkstoffverabreichung in der Kosmetik.

Dadurch, daß erfindungsgemäß die therapeutisch erforderlichen Wirkstoff-Abgabeflächen abwechselnd mit Haftklebeflächen in einer Ebene liegen, kann eine zufriedenstellende lang andauernde innige Kontaktierung des Wirkstoffabgabereservoirs mit der den Wirkstoff aufnehmenden Hautfläche erfolgen, wodurch alleine die kontinuierliche Wirkstoffzufuhr, wie sie therapeutisch erwünscht ist, gewährleistet ist.

Im nachfolgenden soll die Erfindung nun anhand von Ausführungsbeispielen sowie der begleitenden Zeichnung erläutert werden. Dabei zeigen:

Fig. 1    schematisch die Hautkontaktfläche eines Abschnittes eines erfindungsgemäßen flächenförmigen therapeutischen Systems;

Fig. 2    einen Querschnitt gemäß Linie I-I der Fig. 1 in Vergrößerung;

Fig. 3    einen Abschnitt der Hautkontaktfläche einer weiteren bevorzugten Ausführungsform der Erfindung;

Fig. 4    schematisch vergrößert den Querschnitt nach Linie II-II der Fig. 3;

Fig. 5    die Hautkontaktfläche einer pflasterartigen Ausführungsform der Erfindung;

Fig. 6    die Hautkontaktfläche einer weiteren pflasterartigen Ausführungsform der Erfindung; und

Fig. 7    eine Darstellung der in Vitro-Freisetzung von Ketotifen und Bopindolol aus erfindungsgemäßen therapeutischen Systemen.

Erfindungsgemäß erfolgt die Unterteilung der Hautkontaktschicht nach zwei Grundprinzipien:

Prinzip A :

Die Wirkstoffabgabeflächen werden vereinzelt in einer ggf. ebenfalls wirkstoffhaltigen Hautadhäsionsfläche verteilt.

Prinzip B:

Eine Wirkstoffabgabefläche wird durch eine ggf. ebenfalls wirkstoffhaltige Hautadhäsionsfläche unterbrochen.

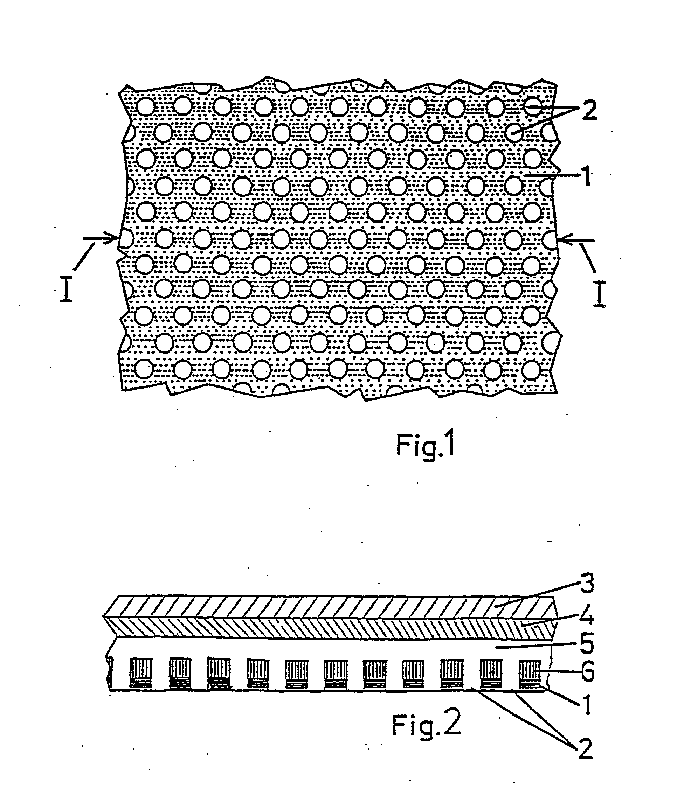

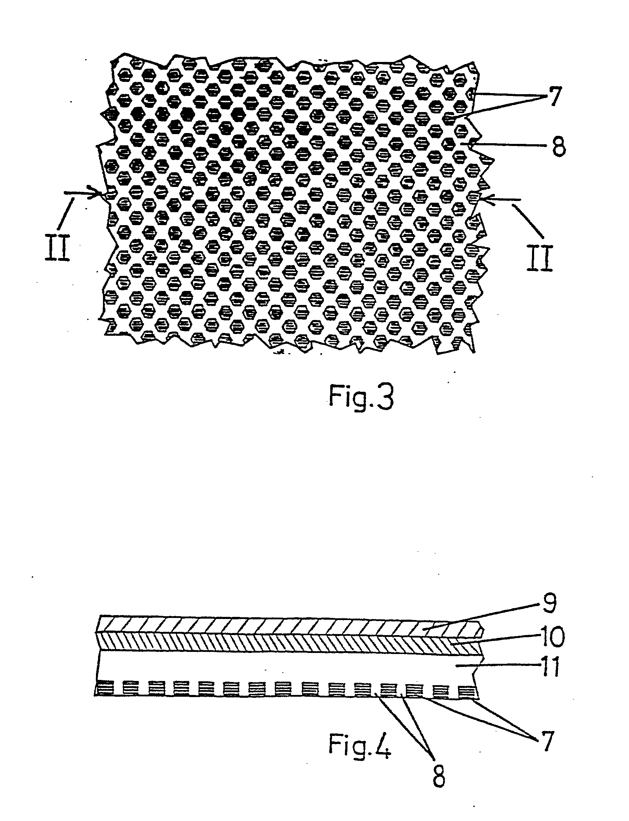

Ausführungsformen der Erfindung gemäß Prinzip A sind in den Figuren 1, 2 und 6 dargestellt; während in den Figuren 3, 4 und 5 Ausführungsformen, die dem Prinzip B entsprechen, gezeigt sind.

In den Figuren 1 und 2 sind in einer haftklebenden Hautadhäsionsfläche 1 kreisrunde Wirkstoffabgabeflächen 2 symmetrisch verteilt. Selbstverständlich soll diese Ausführungsform lediglich beispielhaft genommen und nicht begrenzend für den Erfindungsgedanken gesehen werden, denn die Anordnung der Wirkstoffabgabeflächen, ihre Größe, ihre geometrische Form sowie ihr Abstand voneinander sind variabel und hängen von den therapeutischen und physikalischen Erfordernissen ab.

In Fig. 2 ist der Schnitt entlang der Linie I-I in Fig. 1 dargestellt. Unter einer Rückschicht 3 ist eine Haftklebeschicht 4 angeordnet, die den Zusammenhalt mit einer Wirkstoffreservoirschicht 5 sicherstellt. Die Reservoirschicht 5 ist von einer perforierten Stützschicht 6 überlagert, die hautseitig eine Hautadhäsionsschicht 1 aufweist. Die durch Perforation entstandenen Hohlräume werden vollständig vom Material des Wirkstoffreservoirs 5 ausefüllt, welches hier auch die Wirkstoffbgabeabschnitte 2 bildet, so daß sich an der Hautkontaktfläche Wirkstoffabgabeflächen 2 und Hautadhäsionsabschnitte 1 auf gleichem Niveau abwechseln.

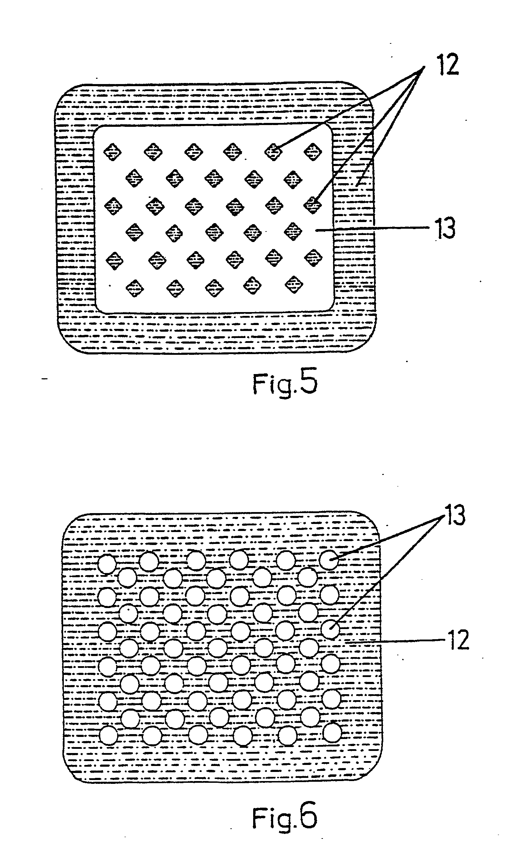

In Fig. 6 ist eine pflasterartige Ausführungsform des Prinzips A gezeigt, wobei vereinzelte Wirkstoffabgabeflächen 13 im Mittelbereich eines ansonsten haftklebenden pflasterartigen Abschnitts erfindungsgemäß als kreisförmige Punkte symmetrisch verteilt sind. Im Randbereich ist ein umlaufender, ununterbrochener klebender Adhäsionsrand 12 vorgesehen, der bei Applikation des Pflasters eine sichere Randverklebung mit der Haut gewährleistet.

Die Figuren 3 und 4 stellen bevorzugte Ausführungsformen des Prinzips B dar. Dabei ist eine Wirkstoffabgabefläche 8 durch symmetrisch angeordnete sechseckige Hautadhäsionsabschnitte 7 unterbrochen. Auch hier sind die Anordnung der Klebebereiche, der Abstand voneinander, ihre Größe und ihre geometrische Form variabel und können entsprechend herstellungstechnischen Notwendigkeiten abgeändert werden. Der Aufbau des in Fig. 3 von der Hautkontaktfläche aus gesehenen Systems geht aus dem in Fig. 4 gezeigten, stark vergrößerten Querschnitt entlang der Linie II-II hervor. Dabei grenzt eine Rückschicht 9 an eine haftgrenzende Zwischenschicht 10 an, die ihrerseits mit einer Reservoirschicht 11 in Kontakt steht. Gewissermaßen eingelassen in die Reservoirschicht 11 sind Hautadhäsionspunkte 7, die mit der Oberfläche des Wirkstoffreservoirs 8 zur Haut hin in einer Ebene liegend abschließen. Die Klebepunkte 7 sind dabei ringsum von der Wirkstoffabgabefläche 8 umgeben.

In Fig. 5 ist ein weiteres Ausführungsbeispiel des Prinzips B der Erfindung dargestellt, in Form eines applikationsfertigen pflasterförmigen Abschnittes. Die Hautadhäsionsabschnitte sind dabei mit 12 und die Wirkstoffabgabeflächen mit 13 bezeichnet. Die Wirkstoffabgabefläche ist bei dieser Ausführungsform symmetrisch mit quadratischen Hautadhäsionspunkten durchsetzt und allseitig von einem umlaufenden Hautadhäsionsrand umgeben. Auch hier ist es besonders vorteilhaft, daß Randklebeprobleme der erfindungsgemäßen pflasterartigen Systeme dadurch vermieden werden können.

Die neuen flächenförmigen therapeutischen Systeme gemäß der Erfindung sind in ihren Applikationsmöglichkeiten sehr variabel, da sie - abgesehen von speziellen Ausführungsformen, wie sie beispielsweise in den Figuren 5 und 6 dargestellt sind - als rapportfreie Bahnware hergestellt und damit in fast beliebige Zuschnitte zerteilt werden können. So kann die Applikation beispielsweise als Pflaster, Streifen oder sogar als Binde erfolgen. Bei willkürlichem Zerteilen der Bahnen ist es möglich, daß Schnittränder entstehen, die nicht vollständig mit Haftkleber belegt sind. Es sollte daher bevorzugt darauf geachtet werden, daß die Schnittlinien stets durch derartige haftklebende Streifen, Ringe o.ä. führen.

Die hautadhäsionsmaterialfreien Abschnitte können durch geeignete Wahl des geometrischen Designs des Systems so klein gehalten werden, daß auch bei längerer Tragedauer der Pflaster keine Ablösung vom Rande her auftritt. Die Fläche der tolerierbaren hautadhäsionsmaterialfreien Abschnitte hängt von der Wahl des Hautadhäsionsmaterials ab. Einen Sonderfall stellt in diesem Zusammenhang ein therapeutisches System dar, bei dem die Wirkstoffabgabefläche streifenförmig in der Hautadhäsionsfläche bzw. die Hautadhäsionsfläche streifenförmig in einer Wirkstoffabgabefläche verteilt ist. Hier darf der Schnitt nur parallel zur Streifenrichtung innerhalb eines haftklebenden Streifens liegen, um einen in dieser Richtung völlig klebefreien Rand zu vermeiden.

Das neue System eignet sich insbesondere zur Verabreichung von medizinischen oder kosmetischen Wirkstoffen an die Haut, die aus haftklebehaltigen Systemen nicht oder nur mit Schwierigkeiten applizierbar sind. Es können aber auch alle einfach zu applizierende Wirkstoffe eingesetzt werden, die alleine oder mit Hilfsmitteln in die Haut zu wandern vermögen.

Es hat sich bereits gezeigt, daß das System besonders zur Verabreichung von Wirkstoffen, wie beispielsweise Ketotifen oder Bopindolol, geeignet ist.

Die Wirkstoffreservoirschicht kann in an sich bekannter Weise hergestellt sein, beispielsweise durch Lösen des Wirkstoffes in einer Matrix, Dispergieren des Wirkstoffes in einer Matrix oder Verteilen des Wirkstoffes in einer Matrixin mikroverkapselter Form; Verteilen des an einem inerten Träger adsorbierten Wirkstoffes in einer Matrix. Selbstverständlich kann das Wirkstoffreservoir auch aus reinem Wirkstoff oder im wesentlichen reinem Wirkstoff bestehen. Die Matrix selbst kann aus nieder- oder hochmolekularen, natürlichen oder synthetischen Stoffen bestehen, während sich ihre Auswahl nach den Eigenschaften des zu verabreichenden Wirkstoffes und den therapeutischen Erfordernissen, wie sie dem Fachmann auf diesem Gebiet bekannt sind, richtet. Im Zusammenhang mit Ketotifen und Bopindolol hat sich beispielsweise eine Matrix auf Basis von wasserquellbaren Polyacrylaten besonders bewährt.

Die Wirkstoffreservoirschicht kann neben den Matrixbestandteilen und Wirkstoffen weitere geeignete, dem Fachmann aufgrund seines Fachwissens bekannte bzw. naheliegende Zusätze, wie beispielsweise Lösungsvermittler, Weichmacher, Stabilisatoren, Füllstoffe und Enhancer enthalten. Die Dicke der Reservoirschicht, d.h. der Abstand zwischen Stützschicht ggf. haftklebend ausgerüsteter Rückschicht wird hauptsächlich durch die therapeutischen Anforderungen an das System bestimmt; es ist bevorzugt, daß die Gesamtdicke des Systems zwischen 40 <m und 5 mm, bevorzugt zwischen 80 <m und 1,2 mm, liegt. In Sonderfällen besteht das Reservoir nur aus der vollständigen oder teilweisen Füllung der Porenöffnungen im Laminat aus Stütz- und Hautadhäsionsschicht, so daß die haftklebend ausgerüstete Rückschicht in direktem Verbund mit der Stützschicht steht. Auch bei diesen Varianten liegen die Wirkstoffabgabeflächen in einer Ebene mit den Hautadhäsionsflächen, wie dies erfindungsgemäß gefordert wird.

Die das Reservoir an der hautabgewandten Seite abdeckende Rückschicht kann durchlässig oder undurchlässig sein. Sie muß flexibel sein und dient in erster Linie zur weiteren mechanischen Stabilisierung des Systems. Sind Teile des Reservoirs oder der eingearbeiteten Wirkstoffe flüchtig - so muß die Rückschicht für diese Stoffe undurchlässig sein. Sie kann ein- oder mehrschichtig sein. Substanzen, die zu ihrer Herstellung verwendet werden können, sind polymere Substanzen, wie beispielsweise Polyethylen, Polypropylen, Polyethylenterephthalat und Polyamide. Als weitere Materialien können auch Metallfolien, wie beispielsweise Aluminiumfolie, allein oder mit einem polymeren Substrat beschichtet, eingesetzt werden. Durchlässige Rückschichten sind beispielsweise textile Flächengebilde, wie Gewebe und Vliesstoffe oder auch poröse Polymer-Materialien. Die erforderliche Dicke der Rückschicht ist vom gewählten Material abhängig, sollte aber im Bereich von 5 bis 150 Mikrometern, bevorzugt zwischen 10 und 60 Mikrometern liegen. Falls die Eigenklebrigkeit der Reservoirschicht für einen dauerhaften Verbund mit der Rückschicht nicht ausreicht, wird der Verbund mit Hilfe einer haftklebenden Zwischenschicht bewerkstelligt, wobei die Haftkräfte zwischen Rück- und Zwischenschicht größer sein müssen als die zwischen Hautkontaktfläche und der Haut. Dazu sind alle physiologisch unbedenklichen, gegenüber Wirkstoffen und übrigen Bestandteilen des Reservoirs inerten Haftkleber geeignet, beispielsweise auf Basis von Kautschuk, kautschukähnlichen synthetischen Homo-, Co-oder Blockpolymeren, Polyacrylsäure, Estern und deren Copolymerisate, Polyurethane und Silicone. Die Dicke der haftklebenden Zwischenschicht 10 liegt bevorzugt zwischen 10 und 100 <m und besonders bevorzugt zwischen 20 und 40 <m.

Bei Ausführungsformen gemäß der Erfindung gemäß Prinzip A - bei denen Wirkstoffabgabeflächen in einer Hautadhäsionsschicht angeordnet werden - hat es sich als vorteilhaft erwiesen, wenn die Adhäsionsschicht auf einer Stützschicht aufgebracht ist, die mit dem Wirkstoffreservoir in Kontakt steht. Anordnungen ohne diese Stützschicht sind natürlich möglich und können bei besonders haltbaren Wirkstoffreservoirschichten vorteilhaft sein. Die Stützschicht weist Öffnungen auf, bevorzugt Durchgangsporen, die bis zur Hautkontaktfläche ganz oder teilweise mit einer Wirkstoffabgabemasse angefüllt sind. Als Materialien für diese flexible Stützschicht kommen beispielsweise Papier, Kunststoff und Metall-Folien oder textile Flächengebilde in Frage. Die Schichtdicke sollte zwischen 5<m und 2 mm, bevorzugt zwischen 10<m und 500 <m liegen. Wenn ein sicherer Verbund zwischen Wirkstoffreservoir- und Stützschicht nicht durch die Eigenklebrigkeit des Reservoirs gewährleistet sein kann, kann er durch Anbringen einer zusätzlichen Haftklebeschicht zwischen Stützmaterial und Wirkstoffreservoirschicht herbeigeführt werden, wobei die Haftkräfte innerhalb des Gesamtsystems größer sein müssen als die zwischen Hautkontaktfläche und Haut. Die Haftklebeschicht kann die gesamte Kontaktfläche zwischen Wirkstoffreservoir- und Stützschicht bedecken oder aber auf die zur Haut parallelen Flächen der Stützschicht beschränkt sein. Der dabei zu verwendende Haftkleber muß, wie der Haftkleber der Zwischenschicht zwischen Reservoir und Rückschicht eine größere Adhäsion innerhalb des Systems als zwischen Haut und Hautkontaktfläche bewirken. Die Dicke dieser Haftklebeschicht bewegt sich bevorzugt zwischen 10 und 100<m, besonders bevorzugt zwischen 20 und 40 <m.

Zum Schutz der Hautkontaktfläche und ggf. auch zur Vermeidung des Entweichens von Bestandteilen des Wirkstoffreservoirs an den offenliegenden Flächen der Wirkstoffabgabefläche wird das erfindungsgemäße System auf der Hautkontaktseite durch eine vor Applikation ablösbaren Schicht abgedeckt. Sie kann aus den gleichen Materialien, wie sie zur Herstellung der Rückschicht eingesetzt werden, hergestellt sein, vorausgesetzt, daß sie ablösbar gemacht werden, beispielsweise durch Aufbringen einer Siliconschicht. Andere ablösbare Schutzschichten, wie sie dem Fachmann allgemein bekannt sind, sind beispielsweise Polytetrafluorethylen, behandeltes Papier, Zellophan, Polyvinylchlorid uä. Zur leichteren Ablösbarkeit kann die Schutzschicht in an sich bekannter Weise mit Abziehhilfen versehen sein. Die Dicke der Schutzschicht ist nicht wesentlich, da sie wegen des Entfernens vor der Applikation den Tragekomfort nicht beeinflußt. Sinnvollerweie kann sie zwischen 10 bis 500 <m, bevorzugt zwischen 20 und 150 <m liegen; sie kann aber auch sehr steif - um Knicken des Systems vor der Aufbringung zu vermeiden - ausgebildet sein.

Das erfindungsgemäße Verfahren zur Herstellung des neuen therapeutischen Systems ist eine Kombination an sich bekannter Verfahrensschritte. Zur Herstellung eines erfindungsgemäßen therapeutischen Systems gemäß dem Prinzip A - vereinzelte Wirkstoffabgabeflächen in einer Adhäsionsfläche - wird eine abweisende Unterlage mit Hautadhäsionsmaterial beschichtet und in einem zweiten Schritt die Stützschicht zukaschiert. Das derart erhaltene Laminat wird anschliessend mit bekannten Verfahren, beispielsweise durch Stanzen oder mit Hilfe einer Stachel- oder Rasterwalze, perforiert, wobei die geometrisch angestrebten Öffnungen durch die Wahl des Werkzeuges festgelegt werden können. Bei diesem Perforationsvorgang, der ggf. auch bei erhöhter Temperatur durchgeführt werden kann, werden in jedem Fall Stütz- und Kleberschicht mit perforiert und die abweisende Unterlage angeprägt oder sogar mit perforiert. Letztere wird in einem weiteren Arbeitsschritt abgezogen und durch die wieder ablösbare Schutzschicht ersetzt. Anschliessend wird auf die perforierte Hautadhäsionsschicht-Seite des Laminats die Wirkstoffreservoirschicht in an sich bekannter Weise aufgebracht.

Falls sie als Lösung aufgebracht wird, muß vor weiteren Verfahrensschritten das Lösungsmittel abgedampft werden. Die Konsistenz der für das Wirkstoffreservoir aufgetragenen Masse wird zunächst so eingestellt, daß ihr vollständiges Eindringen in die Öffnungen des Laminates sichergestellt ist. Durch Zukaschieren einer ggf. mit einer Haftklebeschicht ausgerüsteten (entspricht der Zwischenschicht) Rückschicht wird das System fertiggestellt. Das auf diese Art und Weise erhaltene Bahnmaterial kann nun, wie weiter oben beschrieben, unter Beachtung von therapeutischen Erfordernissen, konfektioniert und verpackt werden.

Eine bevorzugte Weiterbildung dieses Verfahrens besteht darin, daß die Wirkstoffreservoirschicht auf einer - ggf. mit einer Haftklebeschicht ausgerüsteten Rückschicht - erzeugt wird und durch Kaschieren unter Druck oder unter Druck und Wärme auf ein Öffnungen aufweisendes Laminat aus Stützschicht und Hautadhäsionsschicht aufgebracht wird.

Ist für den Verbund zwischen Reservoir- und Stützschicht eine zusätzliche Haftklebeschicht notwendig, so wird diese vor Perforation des Laminats aus Stütz- und Hautadhäsionsschicht auf die Stützschicht aufgebracht. Das derart erhaltene Laminat kann direkt oder nach Abdeckung mit einer wieder ablösbaren Hilfsschicht (beispielsweise Siliconpapier) der Perforation zugeführt werden.

Vor Aufbringen oder Zukaschieren der Reservoirschicht wird dann die Hilfsschicht wieder abgezogen.

Bei Verwendung von Stützmaterialien, die von der Herstellung her bereits mit geeigneten Öffnungen versehen sind, wie textile Flächengebilde (beispielsweise Gewebe oder Vliesstoffe) modifiziert man das Herstellungsverfahren insoweit, als beim ersten Schritt das Stützmaterial einseitig (beispielsweise durch Sprühen) oder allseitig (beispielsweise durch Tauchen) mit einem Haftklebebelag versehen wird und auf der wieder ablösbaren Schutzschicht aufgelegt wird. Die weiteren Verfahrensschritte entsprechen den weiter oben dargestellten nach der Perforation, wobei auf einen Austausch der abweisenden Unterlage verzichtet werden kann.

Die Herstellung von flächigen therapeutischen Systemen nach dem Prinzip A ohne Stützmaterialschicht kann erfolgen, indem die Wirkstoffabgabeflächen in der gewünschten Anordnung, beispielsweise mit Hilfe eines Siebdruckverfahrens, auf einer Schutzschicht erzeugt, mit Haftkleber beschichtet und mit der Rückschicht abgedeckt werden.

Flächige Systeme gemäß der Erfindung nach dem Prinzip B - einer von Hautadhäsionsflächenabschnitten unterbrochene Wirkstoffabgabefläche - können gemäß der Erfindung durch die Kombination folgender Schritte hergestellt werden:

Herstellen von Hautadhäsionsflächen in gewünschter Anordnung mit Hilfe beispielsweise eines Siebdruckverfahrens auf einer abweisenden Unterlage, die bevorzugt als Schutzschicht dient; Beschichten mit wirkstoffhaltiger Reservoirmasse; Zukaschieren der ggf. mit einer Haftklebeschicht versehenen Rückschicht und Konfektionierung. Auch hier kann die o.g. Variante des Erzeugens der Reservoirschicht auf einer ggf. mit einer Haftklebeschicht beschichteten Rückschicht ausgewählt werden.

Die in den Figuren 5 und 6 dargestellten bevorzugten Ausführungsformen des erfindungsgemäßen Systems sind ebenfalls nach den beschriebenen Verfahren herstellbar; wobei bei Pflastern gemäß Fig. 5 nur die das Hautadhäsionsmaterial auftragende Vorrichtung (beispielsweise Siebdruckschablone) entsprechend ausgelegt werden muß, während bei Pflastern gemäß der Fig. 6 das Perforationswerkzeug entsprechend strukturiert sein muß. In beiden Fällen muß bei der Konfektionierung der vorgebene Rapport beachtet werden.

Die Erfindung wird nun anhand nachfolgender Beispiele näher erläutert.

Beispiel 1:

Therapeutisches System nach Prinzip A:

Zur Herstellung wird auf ein durch Siliconisierung abweisend ausgerüstetes Papier (Flächengewicht: 95 g/m$^2$) durch Walzenantrag eine Schicht einer Acrylatcopolymer-Lösung ( DUROTAK 280 - 2415, National Starch & Chemical B.V., Niederlande) (Feststoffgehalt: 44%; 93.3 Teile Acrylat-Copolymer + 6,7 Teile Kolophoniummethylester in Ethylacetat) in einer Dicke aufgebracht, daß nach anschliessendem Trocknen bei 65 Grad C. eine Haftklebeschicht mit einem Flächengewicht von 44 g/m$^2$ resultiert. Haftkleberseitig wird dann ein Polyamidspinnvliesstoff mit einem Flächengewicht von 33 g / m$^2$ zukaschiert und das Laminat einer beheizten Rasterwalze eines Prägekalanders zugeführt. Die Rasterwalze ist folgendermaßen ausgelegt: versetzte Reihen von Pyramidenstümpfen in lichten Abstand von 2 mm; lichter Abstand der Stümpfe innerhalb einer Reihe: 2,5 mm; rautenförmige Grundfläche der Stümpfe: 2,7 mm$^2$; rautenförmige Kopffläche der Stümpfe: 0,48 mm$^2$; Höhe der Stümpfe: 0,85 mm. Die Perforation wird bei 280 Grad C. durchgeführt und liefert ein Laminat, bei dem Vliesstoff und Haftklebeschicht in regelmäßigen Anordnungen Perforationen mit einem Gesamtanteil von 9% der Gesamtfläche aufweisen. Das Siliconpapier wird abgezogen und eine siliconisierte Polyester-Folie (Flächengewicht: 145 g/m$^2$) zulaufen gelassen.

Zur Herstellung der Reservoirmasse werden 32,5 Teile eines Copolymerisates auf Basis von Methacrylsäureestern und Dimethylaminoethylmethacrylat (EUDRAGIT E, Fa. Röhm Pharma, BRD) in 32,5 Teilen Methanol gelöst und 30 Teile eines Gemisches Toluol 2-Octyldodecanol (1:1) zugegeben. Anschliessend werden 20 Teile des Wirkstoffes 4-(1-Methyl-4-piperidylinden)-4H-benzo 4,5 cyclohepta 1,2-b thiophen-10 (9H)-on-hydrogenfumarat (Ketotifen-HFU) eingetragen und gelöst. Die Viskosität der Masse wird mit Methylethylketon auf 0,55 dPa.s eingestellt. Der Massenauftrag auf die nicht klebende Seite des oben hergestellten perforierten Vliesstoff-Laminates erfolgt mittels einer Walze in einer Schichtdikke, daß nach anschliessendem Trocknen bei 65 Grad C. eine Reservoirschicht von 44 g/m$^2$ erhalten wird.

Die haftklebend ausgerüstete Rückschicht wird durch Beschichten eines silikonisierten Papiers mit einer Acrylat-Copolymer-Lösung (DUROTAK 280 - 2416, Fa. National Starch & Chemical B.V., Niederlande) in Ethylacetat (Feststoffgehalt: 41%) und Aufkaschieren der nach Trocknung bei 65 Grad C. erhaltene, 30 g/m$^2$ schweren Haftkleberschicht auf eine aluminisierte Polyesterfolie ·(Flächengewicht: 20 g/m$^2$) hergestellt. Anschliessend wird sie dem Laminat aus Reservoirschicht, Vliesstoff und Haftkleberschicht auf der offenen Seite zukaschiert.

In der anschliessenden Konfektionierung werden aus der erhaltenen Bahnware die Pflasterabschnitte so herausgestanzt, daß sie in der Größe den therapeutischen Erfordernissen entsprechen. Durch eine Stanzung der Schutzschicht über Eck kann eine Abziehhilfe für die Schutzschicht geschaffen werden.

Die Verpackung kann beispielsweise in geeigneten Siegelbeuteln erfolgen.

Beispiel 2:

Herstellung eines Bopindolol-HMO-Plasters:

Die Herstellung des therapeutischen Systems erfolgt analog Beispiel 1, bis auf die Herstellung und Zusammensetzung der Wirkstoffreservoirmasse. Sie wird hier durch Aufösen von 30,6 Gewichtsteilen eines Methacrylsäureester/Dimethylaminoethylmethacrylat - Copolymerisates (EUDRAGIT E, Fa. Röhm Pharma, BRD), gelöst in 30,6 Gewichtsteilen Tetrahydrofuran, Zugabe von 25 Gewichtsteilen (±)-1-(tert.-Butylamino)-3-(2-methyl-1-H-in-dol-4-yl) oxy - 2-propanol-benzoat-hydrogenmalonat (Bopindolol-HMO), 3,75 Gewichtsteile Malonsäure sowie 10 Gewichtsteilen 2-Octyldodecanol und Einstellen der Viskosität mittels Methylethylketon auf 0,55 dPa.s hergestellt.

Beispiel 3:

In Vitro-Freisetzung von Wirkstoffen aus dem therapeutischen System:

Je ein 16 m$^2$ großer Stanzling aus der nach Beispiel 1 und 2 hergestellten Bahnware wird als therapeutisches System mit einer einseitig klebenden Kunststoff-Folie auf der Rückschicht verstärkt und in jeweils einem Gefäß bei 37 Grad C. einem Akzeptormedium (80 ml physiologische Kochsalzlösung) ausgesetzt, so daß ein enger Kontakt mit den Gefäßwänden vermieden und die Freigabeseite ständig vollständig benetzt ist. Nach 2, 4, 8 und 24 Stunden nach Beginn der Insertion des Stanzlings in die physiologische Kochsalzlösung wird das Akzeptormedium erneuert. Die quantitative Bestimmung der freigesetzten Wirkstoffe erfolgt fotometrisch. Die Ketotifen-HFU-Konzentration wird durch Messung der UV-Absorption bei 265 nm und bei Bopindolol-HMU durch Messung der UV-Absorption bei 265 nm bestimmt.

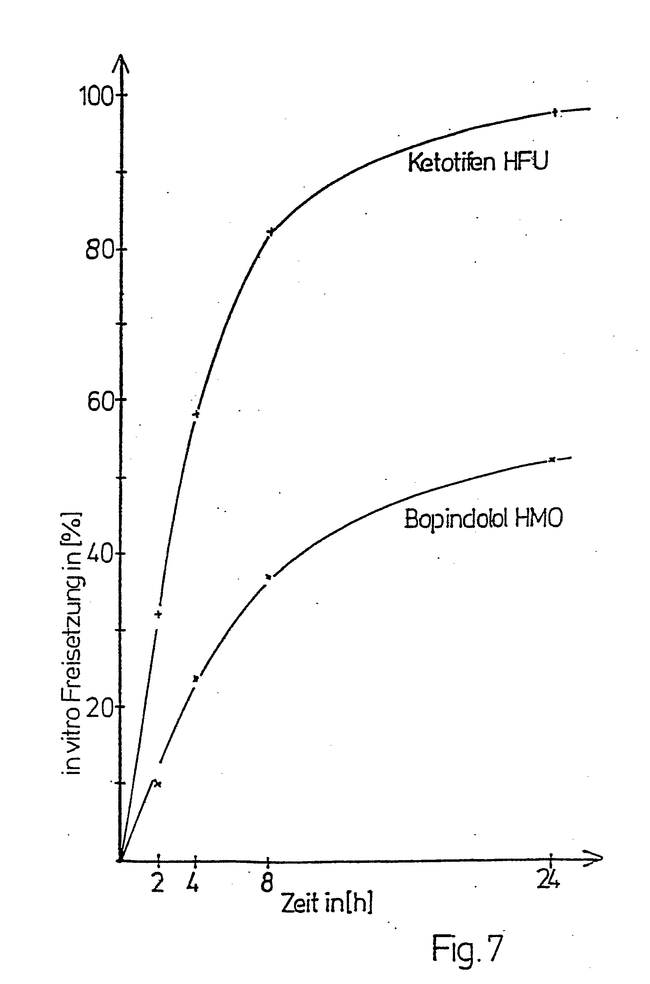

Die Ergebnisse dieser fotometrischen Untersuchung sind in Fig. 7 dargestellt, wobei die freigesetzte Wirkstoffmenge in % über die Zeit aufgetragen ist. Es zeigt sich, daß die Freigabekinetik für beide Wirkstoffe gleich ist, allerdings werden aufgrund der unterschiedlichen physikalischchemischen Eigenschaften von Ketotifen-HFU und Bopindolol-HMO quantitativ unterschiedliche Wirkstoffmengen in Abhängigkeit von der Zeit freigesetzt. Beiden Kurven zeigen aber eindeutig, daß über 24 Stunden eine gesteuerte Wirkstoff-Freisetzung vorliegt.

Beispiel 4:

Ketotifen-haltiges System gemäß Prinzip B der Erfindung:

Eine beidseitig unterschiedlich silikonisierte Polyesterfolie mit einem Flächengewicht von 145 g/m$^2$ wird im Siebdruckverfahren mit einer wässrigen Dispersion eines carboxyl-gruppenhaltigen Acrylat-Copolymeren (ACRONAL 80D, BASF, Ludwigshafen) (Feststoffgehalt: 55%), das mit einem Verdickungsmittel auf Diurethanbasis auf eine Viskosität von 8 Pa.s eingestellt worden ist, beschichtet. Die Siebtrommel ist mit Öffnungen von 1,5 mm Durchmesser versehen, die einen mittleren Abstand von 3,7 mm aufweisen und zu einer offenen Fläche der Siebtrommel von 16,7 % führen. Auf der Folie entstehen analog zur Siebtrommel angeordnete Klebstoffpunkte, die nach Trocknung bei 65 Grad C. ein Flächengewicht von 30 g/m$^2$ besitzen. Bei einer weiteren Beschichtung des Produktes durch Walzenantrag wird die in Beispiel 1 beschriebene Ketotifen-haltige Reservoirmasse aufgetragen. Das Verfahren wird wie in Beispiel 1 weitergeführt.

Beispiel 5 :

Herstellung eines Bopindolol-Systems gemäß Prinzip B der Erfindung:

Wie in Beispiel 3 beschrieben, wird ein erfindungsgemäßes therapeutisches System hergestellt, wobei die in Beispiel 2 beschriebene Wirkstoffreservoirmasse aufgetragen wird.

Beispiel 6:

Herstellung eines therapeutischen Systems mit unterschiedlichen Wirkstoffen in Hautadhäsionsabschnitten und Wirkstoffabgabeabschnitten

Es wird ein wirkstoffhaltiges System, wie in Beispiel 4 beschrieben, hergestellt, wobei lanata-$\beta$-Acetyldigoxin im Acrylatsystem als Reservoirmasse eingesetzt ist und Nitroglyzerin im Hautadhäsionsabschnitt.

## Patentansprüche

1. Flächenförmiges therapeutisches System zur Verabreichung von Wirkstoffen an die Haut, mit Wirkstoff-Reservoir(s) (5) mit Wirkstoffabgabeflächen (2, 8, 13) und mit diesen auf gleichem Niveau liegender hautseitig angeordneter Hautadhäsionsfläche (1, 7, 12), dadurch **gekennzeichnet**, daß entweder die Hautadhäsionsfläche in unterbrochener Weise über die gesamte Hautkontaktfläche verteilt ist und die Unterbrechungen durch Wirkstoffabgabeflächenabschnitte gebildet sind, oder die Wirkstoffabgabefläche in unterbrochener Weise über die gesamte Hautkontaktfläche verteilt ist und die Unterbrechungen durch Hautadhäsionsflächenabschnitte gebildet sind.

2. Flächenförmiges therapeutisches System gemäß Anspruch 1, dadurch gekennzeichnet, daß die Wirkstoffabgabeflächenabschnitte (2, 8, 13) gleichmäßig oder ungleichmäßig in der Hautkontaktfläche verteilt sind.

3. Flächenförmiges therapeutisches System gemäß den Ansprüchen 1 oder 2, dadurch gekennzeichnet, daß die Wirkstoffabgabeflächenabschnitte (2, 13) rund oder eckig sind.

4. Flächenförmiges therapeutisches System gemäß Anspruch 1, dadurch gekennzeichnet, daß in der Hautkontaktfläche Hautadhäsionsflächenabschnitte (7, 12) regelmäßig oder unregelmäßig verteilt sind.

5. Flächenförmiges therapeutisches System gemäß Anspruch 1, dadurch gekennzeichnet, daß die Hautadhäsionsflächenabschnitte (7, 12) rund oder eckig sind.

6. Flächenförmiges therapeutisches System gemäß Anspruch 1, dadurch gekennzeichnet, daß Hautadhäsions- und Wirkstoffabgabeflächenabschnitte streifenförmig alternierend angeordnet sind.

7. Flächenförmmigestherapeutisches System gemäßden Ansprüchen 1 bis 5, dadurch gekennzeichnet, daß die Hautkontaktfläche einen umlaufenden Hautadhäsionsrand aufweist.

8. Flächenförmiges therapeutisches System gemäß einem der vorhergehenden Ansprüche, dadurch gekennzeichnet, daß es ferner eine hautabgewandte flexible Rückschicht (9) und gegebenfalls eine ablösbare Schutzschicht aufweist.

9. Flächenförmiges therapeutisches System gemäß einem der vorhergehenden Ansprüche, dadurch gekennzeichnet, daß es eine haftklebende Zwischenschicht (4, 10) zwischen Rückschicht (9) und Wirkstoffreservoir (11, 5) aufweist.

10. Flächenförmiges therapeutischesSystem gemäßeinem der vorhergehenden Ansprüche, dadurch gekennzeichnet, daß es eine Öffnungen aufweisende Stützschicht (6) in Kontakt mit dem Wirkstoffreservoir (5) aufweist.

11. Flächenförmiges therapeutisches System gemäß Anspruch 10 oder 11, dadurch gekennzeichnet, daß die Stützschicht (6) im wesentlichen aus Papier, einem texilen Flächengebilde, einer einer Metall- oder einer Kunststoff-Folie oder aus Laminaten derselben, besteht.

12. Flächenförmiges therapeutisches System gemäß Anspruch 10 oder 11, dadurch gekennzeichnet, daß sich zwischen Stützschicht (6) und Wirkstoffreservoir ganz oder teilweise eine Haftklebeschicht befindet.

13. Flächenförmiges therapeutisches System gemäß einem oder mehrerender vorhergehenden Ansprüche, dadurch gekennzeichnet, daß das Wirkstoffreservoir einen oder mehrere Wirkstoff(e) mit topischer oder systemischer Wirkung aufweist.

14. Flächenförmiges therapeutisches System gemäß Anspruch 12, dadurch gekennzeichnet, daß mindestens ein Wirkstoff ausgewählt ist aus der Gruppe der Herzglykoside, der Vasodilatatoren, der musculotropen Spasmolytika, der Coronartherapeutica; der Coronardilatatoren, der Antihistaminica, der Analeptica, Analgetica, Antipyretica, einem Hypnotica, Anaesthetica, Antiseptica, einem musculotropen Vasodilatator, Lipidsenker oder einem Vitamin B6; Broncholytica, Cardiaca, Diuretica, Phosphordiesterasehemmer, Theophyllin, dessen Verbindungen und Derivate und Rubefazientes, auch in Form von freien Basen, Salzen oder deren Derivate.

15. Flächenförmiges therapeutisches System gemäß Anspruch 14, dadurch gekennzeichnet, daß der/die Wirkstoff/e ausgewählt ist aus der Gruppebestehend aus Amphetaminil, Betahistin, Beta-Acetyldigoxin, Bopindolol, Buprenorphin, Clemastin, Diclofenac, Diltiazen, Dimenhydrinat, Diethylaminsalicylat, Ethylenglykol-Monosalicylat, 5-Fluorouracil, Glibenclamid, Hydromorphon, Ibuprofen, Isopropyl-4-(2,1,3-benzoxydiazol-4-yl)-1,4-dihydro-5-methoxycarbonyl-2,6-dimethyl-3-pyridin carboxylat, Ketotifen, L-Thyroxin, Nicotin, Nicotinsäure-$\beta$-butoxyethylester, Nonylsäurevanillylamid, Pindolol, Salbutamol, Tamoxifen, Tizanidin, Theophyllin.

16. Flächenförmiges therapeutisches System gemäß einem der Ansprüche 14 oder 15, dadurch gekennzeichnet, daß es eine Kombination von: einem Herzglycosid aus Digitalis lanata-$\beta$-Acetyldigoxin in Kombination mit: einem Vasodilatator, einem musculotropen Spasmolytikum, einem Coronartherapeuticum oder einem Coronardilatator aufweist.

17. Flächenförmiges therapeutisches System gemäß einem der Ansprüche 14 oder 15, dadurch gekennzeichnet, daß es eine Kombination von: einem Antihistaminicum mit einem Glucocorticoid, bevorzugt von Clemastin mit Dexamethason oder Clocortolon-21-pivalat oder einem Antihistaminicum, bevorzugt Antiemeticum-Dimenhydrinat mit einem Vasodilatator, einem Analepticum, einem Analgeticum, einem Hypnoticum, einem musculotropen Vasodilatator oder einem Vitamin B6, aufweist.

18. Flächenförmiges therapeutisches System gemäß einemder Ansprüche 14 oder 15, dadurch gekennzeichnet, daß es eine Kombination von: einem Broncholytikum, Cardiacum, Diureticum, Phosphordiesterasehemmer, wie Theophyllin, mit anderen Theophyllin-Verbindungen oder Theophyllin-Derivaten, aufweist.

19. Flächenförmiges therapeutisches System gemäß einem der Ansprüche 14 oder 15 dadurch gekennzeichnet, daß es ein Rubefaziens, wie Nicotinsäure-$\beta$-butoxyethylester und/oder Nonylsäurevanillylamid in Kombination mit einem Antiphlogisticum, wie bspw. Ethylenglykolmonosalicylat oder Diethylaminsalicylat, aufweist.

20. Flächenförmiges therapeutisches System gemäß einem der vorhergehenden Ansprüche, dadurch gekennzeichnet, daß mindestens ein erster Wirkstoff im Wirkstoffreservoir und ein oder mehrere weitere Wirkstoffe in dem (den) Hautadhäsionsflächenabschnitt(en) vorliegen.

21. Flächenförmiges therapeutisches System gemäß einem der Ansprüche 1 bis 13, dadurch gekennzeichnet, daß die Hautadhäsionsabschnitte (1,7,12) Nitroglyzerin als Wirkstoff aufweisen.

22. Verfahren zur Herstellung des flächenförmigen therapeutischen Systems gemäß einem der vorher-gehenden Ansprüche, gekennzeichnet durch: Vorlegen einer abweisenden Unterlage; Erzeugen einer Hautadhäsionsschicht auf der Unterlage; Zukaschieren eines Stützmaterials; Perforation des derart gebildeten Laminats; Austausch der abweisenden Unterlage gegen eine ablösbare Schutzschicht; Beschichtung der schutzschichtfreien Oberfläche mit wirkstoffhaltiger Reservoirmasse; Zukaschieren der ggf. haftklebend ausgerüsteten Rückschicht und Konfektionierung.

23. Verfahren gemäß Anspruch 17, dadurch gekennzeichnet, daß nach der Perforation des Laminats eine Beschichtung mit der wirkstoffhaltigen Reservoirmasse durch Zukaschieren mit einer auf der Rückschicht erzeugten wirkstoffhaltigen Reservoirschicht unter Druck oder unter Druck/Wärme durchgeführt wird.

24. Verfahren zur Herstellung eines flächenförmigen therapeutischen Systems nach den Ansprüchen 1, 3, 4, 5, 6, 7, 8, 9, 11 bis 16, gekennzeichnet durch: Aufbringen von Hautadhäsionsschichtabschnitten auf eine Schutzschicht-Unterlage; Beschichten der Hautadhäsionsschichtabschnitte und Schutzschichtunterlage mit wirkstoffhaltiger Reservoirmasse; Zukaschieren der gegebenenfalls haftklebend ausgerüsteten Rückschicht; und Konfektionierung.

25. Verwendung des flächenförmigen therapeutischen Systems gemäß einem der vorangehenden Ansprüche für lokale oder systemische transdermale Wirkstoffverabreichung in der Kosmetik.

**Claims**

1. Sheet-like therapeutic system for the administration of active substances onto the skin, with active substance reservoir(s) (5) with active substance delivery areas (2, 8, 13) and with skin adhesion area (1, 7, 12) which lies at the same level as the latter and is arranged on the skin side, characterised in that either the skin adhesion area is distributed in interrupted manner over the entire skin contact area, and the interruptions are formed by active substance delivery area sections, or the active substance delivery area is distributed in interrupted manner over the entire skin contact area, and the interruptions are formed by skin adhesion area sections.

2. Sheet-like therapeutic system according to Claim 1, characterised in that the active substance delivery area sections (2, 8, 13) are uniformly or non-uniformly distributed in the skin contact area.

3. Sheet-like therapeutic system according to Claims 1 or 2, characterised in that the active substance delivery area sections (2, 13) are circular or have corners.

4. Sheet-like therapeutic system according to Claim 1, characterised in that skin adhesion area sections (7, 12) are distributed regularly or irregularly in the skin contact area.

5. Sheet-like therapeutic system according to Claim 1, characterised in that the skin adhesion area sections (7, 12) are circular or have corners.

6. Sheet-like therapeutic system according to Claim 1, characterised in that skin adhesion and active substance delivery area sections are arranged in alternate strip form.

7. Sheet-like therapeutic system according to Claims 1 to 5, characterised in that the skin contact area has an encircling skin adhesion margin.

8. Sheet-like therapeutic system according to any of the preceding claims, characterised in that it furthermore has a flexible backing layer (9) facing away from the skin and, where appropriate, a detachable protective layer.

9. Sheet-like therapeutic system according to any of the preceding claims, characterised in that it has an adhesive interlayer (4, 10) between backing layer (9) and active substance reservoir (11, 5).

10. Sheet-like therapeutic system according to any of the preceding claims, characterised in that it has a supporting layer (6) which has openings and is in contact with the active substance reservoir (5).

11. Sheet-like therapeutic system according to Claim 10 or 11, characterised in that the supporting layer (6) consists essentially of paper, a textile sheet structure, a metal foil or a plastic film or of laminates thereof.

12. Sheet-like therapeutic system according to Claim 10 or 11, characterised in that a layer of adhesive is present wholly or partly between supporting layer (6) and active substance reservoir.

13. Sheet-like therapeutic system according to one or more of the preceding claims, characterised in that the active substance reservoir has one or more active substance(s) with topical or systemic action.

14. Sheet-like therapeutic system according to Claim 12, characterised in that at least one active substance is selected from the group of cardiac glycosides, of vasodilators, of musculotropic spasmolytics, of coronary therapeutics; of coronary dilators, of antihistamines, of analeptics, analgesics, antipyretics, a hypnotics, anaesthetics, antiseptics, a musculotropic vasodilator, lipid lowering agent or a vitamin B6, bronchospasmolytics, cardiac agents, diuretics, phosphordiesterase inhibitors, theophylline, the compounds and derivatives thereof, and rubefacients, also in the form of free bases, salts or derivatives thereof.

15. Sheet-like therapeutic system according to Claim 14, characterised in that the active substance or substances is selected from the group consisting of amphetaminil, betahistine, beta-acetyldigoxin, bopindolol, buprenorphine, clemastine, diclofenac, diltiazen, dimenhydrinate, diethylamine salicylate, ethylene glycol monosalicylate, 5-fluorouracil, glibenclamide, hydromorphone, ibuprofen, isopropyl 4-(2,1,3-benzoxydiazol-4-yl)-1,4-dihydro-5-methoxycarbonyl-2,6-dimethyl-3-pyridine-carboxylate, ketotifen, L-thyroxine, nicotine, $\beta$-butoxyethyl nicotinate, nonyl acid vanillylamide, pindolol, salbutamol, tamoxifen, tizanidine, theophylline.

16. Sheet-like therapeutic system according to either of Claims 14 or 15, characterised in that it has a combination of: a cardiac glycoside from Digitalis lanata-$\beta$-acetyldigoxin in combination with: a vasodilator, a musculotropic spasmolytic, a coronary therapeutic or a coronary dilator.

17. Sheet-like therapeutic system according to either of claims 14 or 15, characterised in that it has a combination of: an antihistamine with a glucocorticoid, preferably of clemastine with dexamethasone or clocortolone 21-pivalate or an antihistamine, preferably antiemetic-dimenhydrinate with a vasodilator, an analeptic, an analgesic, a hypnotic, a musculotropic vasodilator or a vitamin B6.

18. Sheet-like therapeutic system according to either of Claims 14 or 15, characterised in that it has a combination of: a bronchospasmolytic, cardiac agent, diuretic, phosphordiesterase inhibitor, such as theophylline, with other theophylline compounds or theophylline derivatives.

19. Sheet-like therapeutic system according to either of Claims 14 or 15, characterised in that it has a rubefacient such as $\beta$-butoxyethyl nicotinate and/or nonyl acid vanillylamide in combination with an antiinflammatory agent such as, for example, ethylene glycol monosalicylate or diethylamine salicylate.

20. Sheet-like therapeutic system according to any of the preceding claims, characterised in that at least a first active substance is present in the active substance reservoir and one or more other active substances are present in the skin adhesion area section(s).

21. Sheet-like therapeutic system according to any of Claims 1 to 13, characterised in that the skin adhesion sections (1, 7, 12) have nitroglycerine as active substance.

22. Process for the production of the sheet-like therapeutic system according to any of the preceding claims, characterised by: provision of a repellent backing; production of a skin adhesion layer on the backing; laminating on a supporting material; perforation of the laminate formed in this way; replacement of the repellent backing by a detachable protective layer; coating of the surface which has no protective layer with active substance-containing reservoir composition; laminating on the backing layer, which has an adhesive finish where appropriate, and making up.

23. Process according to Claim 17, characterised in that, after the perforation of the laminate, a coating with the active substance-containing reservoir composition is carried out by laminating on, under pressure or under pressure/heat, an active substance-containing reservoir layer produced on the backing layer.

24. Process for the production of a sheet-like therapeutic system according to Claims 1, 3, 4, 5, 6, 7, 8, 9, 11 to 16, characterised by: application of skin adhesion layer sections onto a protective layer backing; coating of the skin adhesion layer sections and protective layer backing with active substance-containing reservoir composition; laminating on the backing layer which has an adhesive finish where appropriate; and making up.

25. Use of the sheet-like therapeutic system according to any of the preceding claims for local or systemic transdermal active substance administration in cosmetics.

**Revendications**

1. Système thérapeutique plan pour administrer des principes actifs à la peau, comprenant un ou des réservoirs de principe(s) actif(s) (5) avec des surfaces de distribution des principes actifs (2, 8, 13) et, au même niveau que celles-ci, des couches d'adhésion à la peau (1, 7, 12) agencées côté peau, caractérisé en ce que soit la surface d'adhésion à la peau est répartie de manière interrompue sur toute la surface de contact avec la peau et les interruptions sont formées par des sections de surface libérant les principes actifs, soit la surface de distribution de principe actif est répartie de manière interrompue sur toute la surface de contact avec la peau et les interruptions sont formées par des sections de la surface d'adhésion à la peau.

2. Système thérapeutique plan selon la revendication 1, caractérisé en ce que les sections de surface libérant les principes actifs (2, 8, 13) sont réparties de manière régulière ou irrégulière dans la surface en contact avec la peau.

3. Système thérapeutique plan selon la revendication 1 ou 2, caractérisé en ce que les sections de surface libérant les principes actifs (2, 13) sont rondes ou polygonales.

4. Système thérapeutique plan selon la revendication 1, caractérisé en ce que les sections de surface d'adhésion à la peau (7, 12) sont réparties de manière régulière ou irrégulière dans la surface en contact avec la peau.

5. Système thérapeutique plan selon la revendication 1, caractérisé en ce que les sections de surface d'adhésion à la peau (7, 12) sont rondes ou polygonales.

6. Système thérapeutique plan selon la revendication 1, caractérisé en ce que les sections de surface adhérant à la peau et libérant les principes actifs sont agencées de manière alternée sous la forme de bandes.

7. Système thérapeutique plan selon les revendications 1 à 5, caractérisé en ce que la surface en contact avec la peau présente un bord adhérant à la peau périphérique.

8. Système thérapeutique plan selon l'une quelconque des revendications précédentes, caractérisé en ce qu'il présente par ailleurs une couche postérieure (9) flexible opposée à la peau et également une couche de protection détachable.

9. Sytème thérapeutique plan selon l'une quelconque des revendications précédentes, caractérisé en ce qu'il présente une couche intermédiaire autocollante (4, 10) entre la couche postérieure (9) et le réservoir de principe actif (11, 5).

10. Système thérapeutique plan selon l'une quelconque des revendications précédentes, caractérisé en ce qu'il présente une couche d'appui (6) présentant des ouvertures en contact avec le réservoir de principe actif (5).

11. Système thérapeutique plan selon la revendication 10 ou 11, caractérisé en ce que la couche d'appui (6) est constituée en substance de papier, d'un tissu textile plan, d'une feuille métallique ou d'une feuille de matière plastique ou encore de stratifié de ces matières.

12. Système thérapeutique plan selon la revendication 10 ou 11, caractérisé en ce qu'il y a entre la couche d'appui (6) et le réservoir de principe actif une couche autocollante qui s'étend sur la totalité ou sur une partie de la surface.

13. Système thérapeutique plan selon une ou plusieurs des revendications précédentes, caractérisé en ce que le réservoir de principe actif présente un ou plusieurs principes actifs à action topique ou systémique.

14. Système thérapeutique plan selon la revendication 12, caractérisé en ce que l'on choisit au moins un principe actif dans le groupe comprenant des glycosides cardiaques, des vasodilatateurs, des spasmolytiques musculotropes, des thérapeutiques coronaires; des dilatateurs coronaires, des antihistaminiques, des analeptiques, des analgésiques, des antipyrétiques, des hypnotiques, des anesthésiques, des antiseptiques, un vasodilateur muscolotrope, un abaisseur du taux de lipides ou une vitamine B6; des broncholytiques, des produits cardiaques, des diurétiques, des inhibiteurs de phosphorediestérase, la théophylline, ses composés et ses dérivés et des rubéfiants, également sous la forme de bases et d'acides libres ou de leurs dérivés.

15. Système thérapeutique plan selon la revendication 14, caractérisé en ce que le ou les principes actifs sont choisis parmi le groupe constitué par l'amphétaminile, la bêtahistine, la bêta-acétyldigoxine, le bopindolol, la buprénorphine, la clémastine, le diclofénac, le diltiazène, le dimenhydrinate, le diéthylaminsalicylate, monosalicylate d'éthylène glycol, le 5-fluorouracile, le glibenclamide, l'hydromorphone, l'ibuprofène, l'isopropyl-4-(2,1,3-benzoxydiazol-4-yl)-1,4-dihydro-5-méthoxycarbonyle-2,6-diméthyl-3-pyridinecarboxylate, le cétotifène, la L-thyroxine, la nicotine, le butoxyéthylester de l'acide nicotinique, le vanillylamide d'acide nonyllique, le pindolol, le salbutamol, le tamoxifène, la tizanidine, la théophylline.

16. Système thérapeutique plan selon la revendication 14 ou 15, caractérisé en ce qu'il présente une combinaison d'un glycoside cardiaque constitué de digitalis lanata-β-acétyldigoxine avec un vasodilatateur, un spasmolytique musculotrope, un thérapeutique coronaire ou un dilatateur coronaire.

17. Système thérapeutique plan selon la revendication 14 ou 15, caractérisé en ce qu'il présente une combinaison d'un antihistaminique avec du glucocorticoïde, de préférence de la clémastine avec du dexaméthasone ou du clocortolon-21-pivalate ou un antihistaminique, de préférence du dimenhydrinate anti-émétique avec un vasodilatateur, un analeptique, un analgésique, un hypnotique, un vasodilatateur muscolotrope ou une vitamine B6.

18. Système thérapeutique plan selon la revendication 14 ou 15, caractérisé en ce qu'il présente une combinaison d'un broncholytique, d'un cordial, d'un diurétique, d'un inhibiteur de phosphorediestérase comme la théophylline avec d'autres composés de théophylline ou dérivés de théophylline.

19. Système thérapeutique plan selon la revendication 14 ou 15, caractérisé en ce qu'il présente un rubéfiant comme le β-butoxyéthylester de l'acide nicotinique et/ou le vanillylamide de l'acide nonylique en combinaison avec un antiphlogistique, tel que par exemple le monosalicylate d'éthylène glycol ou le diéthylaminesalyicilate.

20. Système thérapeutique plan selon l'une quelconque des revendications précédentes, caractérisé en ce qu'au moins un premier principe actif est présent dans le réservoir de principe actif et un ou plusieurs autres principes actifs sont présents dans le ou les sections de surface adhérant à la peau.

21. Système thérapeutique plan selon l'une quelconque des revendications 1 à 13, caractérisé en ce que les sections d'adhésion à la peau (1, 7, 12) présentent de la nitroglycérine comme principe actif.

22. Procédé de fabrication d'un système thérapeutique plan selon l'une quelconque des revendications précédentes, caractérisé en ce que l'on dispose un support anti-adhérant, on élabore une couche d'adhésion à la peau sur le support, on applique une matière d'appui, on perfore le stratifié ainsi obtenu, on échange le support anti-adhérant précité contre une feuille de protection détachable, on applique sur la surface sans couche de protection une substance réservoir de principe actif, on applique la couche postérieure éventuellement autocollante et on conditionne le tout.

23. Procédé selon la revendication 17, caractérisé en ce que, après la perforation du stratifié, un revêtement avec la substance réservoir de principe actif est réalisé en appliquant une couche-réservoir de principe actif sur la couche postérieure avec application de pression et/ou de pression/chaleur.

24. Procédé de fabrication d'un système thérapeutique plan selon les revendications 1, 3, 4, 5, 6, 7, 8, 9, 11 à 16, caractérisé en ce que l'on applique des sections de couche d'adhésion à la peau sur un support-couche de protection, on revêt les sections de la couche d'adhésion à la peau et le support-couche de protection d'une substance réservoir de principe actif, on applique la couche postérieure éventuellement autocollante et on conditionne le tout.

25. Emploi du système thérapeutique plan selon l'une quelconque des revendications précédentes pour l'administration transdermique locale ou systémique d'un principe actif dans l'industrie des cosmétiques.

Fig.1

Fig.2

Fig.3

Fig.4

Fig.5

Fig.6

Fig. 7